(19) Europäisches Patentamt
European Patent Office
Office européen des brevets

(11) **EP 1 721 036 B1**

(12) **EUROPÄISCHE PATENTSCHRIFT**

(45) Veröffentlichungstag und Bekanntmachung des Hinweises auf die Patenterteilung:
**27.02.2008 Patentblatt 2008/09**

(21) Anmeldenummer: **05707424.7**

(22) Anmeldetag: **16.02.2005**

(51) Int Cl.:
*D04H 1/00* *(2006.01)*   *D04H 1/44* *(2006.01)*
*D04H 1/48* *(2006.01)*

(86) Internationale Anmeldenummer:
**PCT/EP2005/001552**

(87) Internationale Veröffentlichungsnummer:
**WO 2005/080655 (01.09.2005 Gazette 2005/35)**

(54) **VERFAHREN ZUR HERSTELLUNG EINER FASERBAHN AUS CELLULOSEFASERN IN EINEM TROCKENLEGUNGSPROZESS**

METHOD FOR THE PRODUCTION OF A FIBROUS WEB FROM CELLULOSE FIBERS IN A DRAINING PROCESS

PROCEDE POUR REALISER UNE BANDE DE FIBRE DE CELLULOSE PAR ASSECHEMENT

(84) Benannte Vertragsstaaten:
**DE FR IT PL SE**

(30) Priorität: **25.02.2004 DE 102004009556**

(43) Veröffentlichungstag der Anmeldung:
**15.11.2006 Patentblatt 2006/46**

(73) Patentinhaber: **Concert GmbH**
**16928 Falkenhagen (DE)**

(72) Erfinder: **HANSEN, Morten, Rise**
**16928 Pritzwalk (DE)**

(74) Vertreter: **Eisenführ, Speiser & Partner**
**Anna-Louisa-Karsch-Strasse 2**
**10178 Berlin (DE)**

(56) Entgegenhaltungen:
**EP-A- 0 033 988      WO-A-99/25281**
**FR-A- 2 138 296      US-A- 4 292 271**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

**Beschreibung**

Die Erfindung betrifft ein Verfahren zur Herstellung einer Faserbahn aus Cellulosefasern mit absorbierenden Eigenschaften in einem Trockenlegungsprozess. Die Erfindung bezieht sich außerdem auf eine Faserbahn, die nach dem vorgenannten Verfahren hergestellt wurde.

Faserbahnen gemäß Erfindung sollen für die Herstellung von Hygieneartikeln geeignet sein, insbesondere für Inkontinenz-Artikel, Wegwerf-Windeln, Slipeinlagen oder Monatsbinden, wobei sie im Wesentlichen als absorbierende Kerne für die genannten Hygieneartikel dienen. Eine weitere Anwendung stellen Saugeinlagen für Lebensmittelpackungen dar. Weitere Anwendungen, bei denen es auf die Verwendung von Cellulosefasern unter Vermeidung von anderem Fasermaterial ankommt, sind dem Fachmann bekannt.

Die Herstellung von Faserbahnen aus Cellulosefasern in Trockenlegungsprozessen, bei denen anders als im Nasslegungsverfahren die Fasern in einem Luftstrom auf einem Band abgelegt und durch Presswalzen verdichtet werden, ist seit vielen Jahren bekannt.

Unter Cellulosefasern, die für Hygieneprodukte verwendet werden können, werden insbesondere so genannte "fluff pulp"-Fasern verstanden, die aus nordischen Weichhölzern oder aus amerikanischer Southern Pine gewonnen werden. Die mittlere Faserlänge derartiger Cellulosefasern beträgt etwa 2,4 - 2,8 mm. Die Absorptionsfähigkeit der locker verdichteten trockenen Fasern beträgt etwa 10 - 12 g Flüssigkeit pro Gramm Fasern. Entsprechende Daten finden sich in WO 9316228 (Norlander), Tabelle 2, für unbehandeltes Referenzmaterial oder in WO 8804704 (Graef), Tabelle 7. Es ist bekannt, weiteres absorbierendes Material in die Fasermatrix einzubauen; insbesondere sind hierzu so genannte superabsorbierende Polymere (SAP) mit einer Absorptionsfähigkeit wesentlich größer als 10 g Flüssigkeit pro Gramm Polymer, die auch in Faserform (SAF = superabsorbierende Fasern) hergestellt werden, bekannt.

Die US-Patentschrift 3 692 622 beschreibt einen saugfähigen Faserverbund in Form einer Cellulosefaserbahn zur Herstellung von Papiertüchern, wobei die Bahn mit einem vorbestimmten Prägemuster versehen ist. Die Faserbahn ist ein Kontinuum aus trocken gelegten, im Wesentlichen unverbundenen Fasern mit einer Länge von weniger als 1,27 cm, welche durch Prägungen, die 5 bis 40% der Bahnoberfläche bedecken und einen geringeren Abstand voneinander haben als der Länge der einzelnen Fasern entspricht, zu einer in sich zusammenhängenden Bahn derart verbunden sind, dass die Dicke in den nicht geprägten Gebieten mindestens 2,5 mal so groß ist wie in den geprägten Gebieten.

Bei Faserbahnen, die ausschließlich aus Cellulosefasern bestehen, stellt es ein Problem dar, die Saugfähigkeit auf einen hohen Stand zu bringen und gleichzeitig die Staubentwicklung niedrig zu halten. Zwar ist bekannt, Faserbahnen mit anschmelzbaren synthetischen Fasern, auch so genannten Bicomponenten-Fasern, herzustellen und mit niedrigem Staubanteil zu versehen. Für die Erfindung stellt sich jedoch die Teilaufgabe, auf derartige synthetische Faser-Beimischungen zu verzichten. Vielmehr soll erreicht werden, eine Faserbahn aus Cellulosefasern herzustellen, bei denen der Abrieb durch Fusseln, der so genannte Staubbereich, unter 0,1% der Ausgangsbahn liegt. Dabei soll der Einsatz von Latex-Bindern auf einem niedrigen Niveau gehalten werden, das heißt das Flächengewicht im Trockenzustand soll im Bereich kleiner als 5 g/m² liegen.

Diese Aufgabe, d.h. insbesondere die Verhinderung des Faserstaubens "linting", wird gelöst durch ein Verfahren zur Herstellung einer Faserbahn aus Cellulosefasern mit absorbierenden Eigenschaften in einem Trockenlegungsprozess, mit folgenden Verfahrensschritten:

-   Bildung einer im wesentlich gleichmäßigen trockenen Faserlegung aus losen Fasern mit einem geringen, im Bereich der Restfeuchte liegenden Feuchtegehalt,

-   Pressen und Prägen der Faserlegung zu einer Faserbahn unter Ausbildung eines Prägemusters mit verdichteten Faser-Verbundbereichen, in denen die Fasern im Wesentlichen unter Eigenbindung untereinander verbunden sind,

-   Befeuchten der Faserbahn mit einer Wasser-Latex-Mischung mit hohem Wasser-Anteil auf wenigstens einer der Außenbereiche, wobei der Einsatz von Latex-Bindern so bemessen ist, dass das Flächengewicht des Latex im Trockenzustand weniger als 5g/ m² beträgt.

-   Ausfällen des Latex durch Trocknen unter Bindung der Fasern innerhalb und außerhalb der Faser-Verbundbereiche.

Als Cellulosefasern kommen die von Herstellern wie Weyerhaeuser oder Tartas vertriebenen "fluff pulp"-Fasern in Frage, die in verdichteten Rollen angeliefert werden und über Hammermühlen wieder zu einer Faserwatte und zu einzelnen Fasern aufgeschlossen werden. Die Faserlänge der zu Faserbahnen verarbeiteten Fasern liegt je nach Ausgangsmaterial in der Größenordnung 2,0 bis 3,0 mm, vorzugsweise bei 2,3 bis 2,8 mm.

Die Anwendung von Latex zur Bindung von Fasern ist grundsätzlich bekannt. Im Allgemeinen wird Latex nur dazu verwendet, eine Bindung von ungeprägten Cellulose-FaserBahnen zu erreichen. Es hat sich jedoch herausgestellt, dass durch Ausbildung eines Prägemusters mit verdichteten Faser-Verbundbereichen, in denen die Fasern im Wesentlichen unter Eigenbindung untereinander verbunden sind, zusammen mit einer in den Außenbereichen der Faserbahn vorhandenen Latexbindung ausreicht, das Faserstauben, auch "linting" oder Fusseln genannt, wesentlich zu unterdrükken. Unter "Restfeuchte" wird die natürliche Feuchte in den Cellulosefasern nach ihrer Herstellung verstanden.

Unter dem Begriff Eigenbindung ("self bonding") soll die bei Cellulose-Fasern bekannte Neigung verstanden werden, unter hohem Druck und ohne vorher zugeführte Feuchtigkeit eine Bindung einzugehen.

Zur weiteren Verfestigung trägt bei, dass eine Wasser-Latex-Mischung mit vorzugsweise hohem Wassergehalt nach dem Pressvorgang aufgetragen wird, wobei im Allgemeinen ein Gemisch aus 90 bis 99 Gew.-% Wasser mit einem Zusatz von 10 bis 1 Gew.-% Latex verwendet wird. Die Menge des aufgebrachten Wassers ist wichtig, da das Wasser, wenn es verdunstet, Wasserstoffbrücken-Bindungen zwischen den Cellulosefasern erzeugt.

Das Pressen und Prägen der Faserlegung geschieht in einer Presswalzenanordnung. Vorzugsweise ist wenigstens eine der Walzen eine Stachelwalze. Dabei hängt der aufzubringenden Liniendruck ab von der Masse pro Flächeneinheit, den die Faserlegung ausmacht; die Drücke liegen vorzugsweise im Bereich von 30 N/mm bis 120 N/mm.

Die Faserbahn wird mit einem an die Faserbahn angelegten Unterdruck belegt, so dass während und/oder nach dem Befeuchten der Faserbahn mit dem Wasser-Latex-Gemisch die Durchdringung gesteuert werden kann. Je nach eingesetzten Pulpefasern und deren mittlerer Faserlänge und/oder Flächengewicht der Faserbahn ist eine unterschiedliche Eindringtiefe erforderlich.

Um die Absorptionsfähigkeit zu erhöhen, wird der Faserlegung bzw. der Faserbahn Fasern oder Granulat aus superabsorbierenden Polymeren (so genanntes SAP) vor dem Pressen und Prägen beigemischt. Die superabsorbierenden Polymere können unter die abzulegenden Fasern gemischt werden oder unter Schichtbildung in die Faserlegung eingebracht werden.

Als SAP eignen sich insbesondere hydrogel-bildende Polymere, die Alkalimetallsalze folgender organischer Säuren sind: Poly-Acrylsäure), Poly-Methacrylsäure); Copolymere von Acrylsäure und Methacrylsäure mit Acrylamid, Vinylalkohol, Acrylestern, Vinylpyrrolidon, Vinyl-Sulfonsäuren, Vinylacetat, Vinylmorpholinon uns Vinylethern; hydrolysierte, modifizierte Stärke; Maleinanhydrid-Copolymere mit Ethylen, Isobutylen, Styrol und Vinyl; Polysaccharide, wie Carboxymethyl-Stärke, Carboxymethyl-Cellulose und Hydroxypropyl-Cellulose; Polyacrylamide; Polyvinylpyrrolidon; Polyvinylmorpholinon; Polyvinylpyridin; Copolymere und Mischungen der vorstehenden Substanzen. Die hydrogelbildenden Polymere sind vorzugsweise vernetzt, damit sie wasserunlöslich werden. Insbesondere wird ein gering vernetztes Poly-Na-Acrylat verwendet. Das Vernetzen kann durch Bestrahlung induziert werden oder durch covalente, ionische oder van-der-Waals-Kräfte sowie durch Wasserstoff-Brückenbindung hervorgerufen werden. Die superabsorbierenden Polymere können in beliebiger, für die jeweilige Verarbeitungsart geeigneten Form beigemischt werden, also beispielsweise als Granulat, in Faserform, als Fasergruppen (Filamente), in Flockenform, als Kügelchen, Stäbchen oder dergl.

Die Dichte der Faser-Verbundbereiche richtet sich nach der geforderten Reißfestigkeit. Üblicherweise werden 16 bis 49 verdichtete Faser-Verbundbereiche pro $cm^2$ in die Faserbahn eingebracht.

Die verdichteten Faser-Verbundbereiche decken dabei vorzugsweise jeweils eine Fläche von 0,03 bis 1 $mm^2$ ab.

Die Wasser-Latex-Mischung kann mit Hilfe von Walzenbefeuchtung oder durch Aufsprühen auf die Faserbahn aufgebracht werden. Das Abtrocknen des Wassers zum Ausfällen des Latex kann mit Hilfe von Wärmestrahlung oder mittels Durchblasen von Warmluft durch die Faserbahn erfolgen.

Als Latices sind beispielsweise die in der Airlaid-Industrie üblichen Substanzen verwendbar. Als Beispiel sei eine Ethylen-Vinyl-Acetat-Emulsion genannt, die unter dem Handelsnamen AIRFLEX (Hersteller Air Products and Cemicals Inc., Allentown, USA) erhältlich ist. Es können auch biologisch abbaubare Latices, insbesondere ein Latex auf Stärkebasis, verwendet werden. Wird zusätzlich auch das superabsorbierende Polymer auf biologisch abbaubarer Basis hergestellt, so lässt sich die Faserbahn nach der Benutzung in einem Kompostierungsschritt abbauen.

Ein besonderes Augenmerk wird auch auf das Latex gerichtet. Dieser kann von vornherein durch entsprechende Ausrüstung so eingestellt werden, dass es nach dem Ausfällen und Verbinden der Faserbahn eine hydrophile oder hydrophobe Eigenschaft hat. Dabei können für die gegenüberliegenden Seiten der Faserbahn unterschiedliche Latices verwendet werden. Für eine Windel beispielsweise wird auf der körperabgewandten Seite eine hydrophobe Faserbahn verwendet, während für die körperzugewandte Seite eine hydrophile Einstellung gewählt wird.

Die Erfindung bezieht sich auch auf eine mit geringem Latex-Binder-Einsatz hergestellte Faserbahn, bei der der Staubgrad unterhalb 0,1 % liegt, gemessen nach einem standardisierten Verfahren.

Ein standardisiertes Verfahren zur Bestimmung des Staubgehaltes in Faserstoffbahnen, wie es bei der CONCERT GmbH angewandt wird, wird wie folgt dargestellt:

Verwendete Geräte:

Staub-Testgerät mit durchsichtiger, abgeschlossener Kammer, mit rotierender Scheibe mit zwei senkrechten Stäben, Befestigungsklammern für Proben, Motor mit Drehzahl 150 U/min, Timer, Polyesterfolie zum Stützen der Proben; Tisch-Probenschneider; Kurzzeitwecker; Laborhandschuhe und Analysenwaage AG 204 von METTLER.

Testbedingungen:

Die zur Messung verwendeten Proben sollen unter folgenden Umgebungsbedingungen untersucht werden:

Raumtemperatur 23 Grad Celsius $\pm$ 2°C

Relative Feuchte 50% $\pm$ 2%

Prinzip der Bestimmung:

Die zu bestimmenden Proben werden im Testgerät eingespannt und dort eine definierte Zeit geschlagen (Abschütteln des Staubes). Durch Differenzwägung der Probe vor und nach der Prozedur wird der Staubverlust ermittelt und daraus der Staubgehalt in Prozent berechnet.

Durchführung der Bestimmungen:

a) Probenvorbereitung:
Proben zur Staub Messung müssen vor der Bestimmung mindestens zwei Stunden im Labor dem oben angegebenen Raumklima angepasst werden. Da die Proben hygroskopisch sind und Feuchtigkeit aufnehmen, sollten sie, wenn möglich, mit Handschuhen angefasst werden, um Einflüsse auf das Analysenergebnis zu vermeiden.

b) Durchführung der Staubbestimmung bei SAP-freien Airlaid-Typen:

aa) Von der entsprechenden Probenposition werden für eine Analyse je zwei Proben zu 125mm x Slit-Breite (=1 Probeneinheit) geschnitten, ebenso ein gleichgroßes Stück Polyesterfolie.

bb) Die Probeneinheit wird auf der Analysenwaage nach einer Stabilisierungszeit von 20 Sekunden (Kurzzeitwecker) auf 0,0001 g genau gewogen.

cc) Die Probeneinheit ist anschließend in einer Klammer im Staub-Testgerät folgendermaßen einzuspannen: Beide Proben sind deckungsgleich mit den vermeintlich staubigeren Seiten nach außen zusammenzulegen, darauf bzw. dahinter kommt die stützende Polyesterfolie. Diese Einheit ist an den kurzen Seiten ca. 4 bis 5 cm (der Rest ragt nach oben heraus) soweit in der Klammer einzuspannen, dass die Stäbe der rotierende Scheibe die Probeneinheit in der Mitte treffen. Dazu ist die Kammertür zu öffnen und anschließend wieder zu schließen.

dd) Starten des Staubtesters mit dem Schalter nach rechts (Rechtslauf) oder links (Linkslauf) so, dass die Probe (nicht die Folie) zuerst getroffen wird. Das Gerät läuft mit einer Drehzahl von ca. 150 U/min und besitzt einen Timer, der das Gerät nach einer Minute automatisch abstellt. Während dieser Zeit wird die Probe ca. 300 mal geschlagen, so dass vorhandener Staub abfällt.

ee) Nach einer Minute wird die Probe gedreht und mit der anderen Seite in die Klammer eingespannt. Dabei werden auch die Proben gewendet (Außenseite wird Innenseite), und bei der Folie wird die Seite getauscht (vor den Proben, hinter den Proben). Das Gerät wird dann eine weitere Minute angestellt, aber jeweils in der Gegenrichtung.

ff) Anschließend wird die Probeneinheit erneut auf 0,0001 g ausgewogen. Dabei ist die Waage exakt 20 Sekunden lang zu stabilisieren.

c) Durchführung der Staubbestimmung von SAP-haltigen Airlaid-Typen

aaa) Von der entsprechenden Probenposition wird für eine Analyse eine Probe zu 125 mm x Slit-Breite geschnitten, ebenso ein gleichgroßes Stück Polyesterfolie.

bbb) Die Probeneinheit wird auf der Analysenwaage nach einer Stabilisierungszeit von ca. drei Minuten (Kurzzeitwecker) auf 0,0001 g genau gewogen.

ccc) Die Probeneinheit ist anschließend in einer Klammer im Staub-Testgerät folgendermaßen einzuspannen: Beide Proben sind deckungsgleich mit den vermeintlich staubigeren Seiten nach außen zusammenzulegen, darauf bzw. dahinter kommt die stützende Polyesterfolie. Diese Einheit ist mit der kurzen Seite ca. 4 bis 5 cm (der Rest ragt nach oben heraus) soweit in der Klammer einzuspannen, dass die Stäbe der rotierende Scheibe die Proben in der Mitte treffen. Dazu ist die Kammertür zu öffnen und anschließend wieder zu schließen.

ddd) Starten des Staubtesters mit dem Schalter nach rechts (Rechtslauf) oder links (Linkslauf) so, dass die Probe (nicht die Folie) zuerst getroffen wird. Das Gerät läuft mit einer Drehzahl von ca. 150 U/min und besitzt einen Timer, der das Gerät nach einer Minute automatisch abstellt. Während Zeit wird die Probe ca. 300 mal geschlagen, so dass vorhandener Staub abfällt.

eee) Nach einer Minute wird die Probe gedreht und mit der anderen Seite in die Klammer eingespannt. Dabei werden auch die Probe gewendet (Außenseite wird Innenseite), und bei der Folie wird die Seite getauscht (vor den Proben, hinter den Proben). Das Gerät wird dann eine weitere Minute angestellt, aber jeweils in der Gegenrichtung.

fff) Anschließend wird die Probeneinheit erneut auf 0,0001 g ausgewogen. Dabei ist die Waage exakt drei Minuten lang zu stabilisieren.

Jede Analyse wird als Doppel Bestimmung durchgeführt, indem beide Klammern im Staub-Testgerät mit jeweils zwei gleichartigen Proben (Probeneinheiten) genutzt werden.

Mindestens nach jeder vierten Analyse ist der abgeschüttelte Staub im Testgerät zu entfernen.

Berechnung:

$$\texttt{Staub (\%) = (A - B)} \bullet \texttt{100/A}$$

A = Gewicht der Probe vor dem Test

B = Gewicht der Probe nach dem Test

Aus Doppelbestimmungen sind die Mittelwerte zu berechnen.

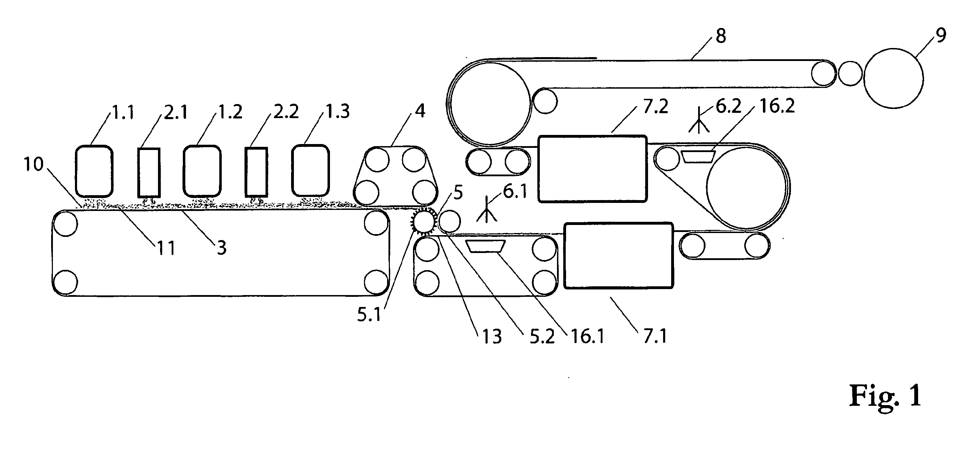

Ein Ausführungsbeispiel des Verfahrensablaufes wird anhand der <u>Figur 1</u> beschrieben.

Von einer Hammermühle (nicht dargestellt) werden Cellulosefasern 10 mit einer mittleren Faserlänge von 2,4 mm von einem ersten Former 1.1 auf einem Förderband 3 als loses Vlies 11 laufend abgelegt, so dass eine Schicht aus wirr durcheinander liegenden Cellulosefasern erzeugt wird. Diese Schicht ist noch weitgehend unverdichtet. Eine weitere vorgeformte Cellulosefaser-Schicht wird durch den Former 1.2 als Schicht abgelegt. Abschließend wird eine dritte Schicht aufgelegt durch den Former 1.3. Die einzelnen Faserlagen können auch unterschiedliche Fasern und differierende Faserdichten umfassen. Außerdem ist möglich, den Fasern superabsorbierende Polymere beizumischen, um die Absorptionsfähigkeit zu erhöhen. Hierbei handelt es sich um handelsübliche Produkte, die im Hygienebereich seit einiger Zeit verwendet werden und die bereits beschrieben wurden. Das Zumischen in Schichtenfolge geschieht über die Vorratsbehälter 2.1 und 2.2. Es kann jedoch das superabsorbierende Polymer auch den Fasern homogen vor dem Aufstreuen beigemischt sein.

Der Feuchtigkeitsgehalt der Faser-SAP-Mischung ist allein durch die so genannte Restfeuchte gegeben. Die Restfeuchte liegt bei natürlichen Fasern, wie sie hier verwendet werden, bei etwa 6 bis 10 Gew.-%. Als SAP werden im beschriebenen Ausführungsbeispiel Natrium-Polyacrylate in vernetzter Form verwendet, wie sie unter dem Markennamen Favor, Hersteller Stockhausen GmbH & Co. KG, marktgängig, insbesondere für Hygieneartikel, auf dem Markt angeboten werden.

Über eine "web transfer" genannte Fördereinrichtung 4 erfolgt eine Vorverdichtung und ein Transport zu einem Walzenpaar 5, das aus einer Prägewalze 5.1 und einer zu dieser anstellbaren Glattwalze 5.2 besteht. Die beiden walzen sind horizontal zueinander ausgerichtet. Das Formerband wird nicht durch die Walzen hindurchgeführt. Das Material der Walzen ist Stahl.

Die Prägewalze 5.1 trägt Prägestacheln, die vergleichsweise steile Flanken haben. Die Höhe der Stacheln liegt zwischen 0,3 und 1,0 mm. Ein hoher Liniendruck zwischen den beiden Druckwalzen sorgt für Höhenunterschiede zwischen den ungeprägten und den geprägten Arealen von wenigstens 1 : 8, bevorzugt wenigstens 1 : 10. Dabei sind je nach Flächengewicht der Faserlegung unterschiedliche Drücke erforderlich.

Die Prägebereiche nehmen etwa 8 bis 40 % der gesamten Bahnoberfläche ein. Zu große Flächenanteile wirken sich nachteilig auf die Saugfähigkeit aus, während zu geringe Flächenanteile die Reißfestigkeit soweit herabsetzen, dass diese nicht mehr ausreicht. Angestrebt wird bei der Bahn eine Reißfestigkeit von wenigstens 15 N pro 50 mm Bahnbreite.

Neben dem Flächenanteil des gesamten Bindungsgebietes ist auch die Bindungsdichte wichtig, die die in einem regelmäßigen Flächenmuster verteilten Faser-Verbindungsbereiche umfassen sollte. Die Abstände zwischen den einzelnen Verbindungsbereichen sollten kleiner als die mittlere Faserlänge sein. Es haben sich 16 bis 49 verdichtete Faser-Verbindungsbereiche pro cm$^2$ der Faserbahn als günstiger Wertebereich erwiesen.

Bei dem Verdichten in den Verbindungsbereichen muss ein ausreichender Druck erzeugt werden, damit die Fasern in den einzelnen Bindungsbereichen eine Eigenbindung eingehen können. Bei Faserlegungen im Bereich 500 g/m$^2$ ist der erforderliche Liniendruck etwa 40 N/mm; bei Faserlegungen im Bereich von 150 g/m$^2$ liegt der Druck bei 110 N/mm.

Durch die vorgenommene Verdichtung erhält die Faserbahn eine hohe Reißfestigkeit und Integrität, das heißt, eine Delamination tritt nicht ein, da in Z-Richtung der Faserbahn ein hoher Zusammenhalt beobachtet wird.

Die geprägte Faserbahn wird anschließend mit einem Wasser-Latex-Gemisch über eine Flüssigkeitssprühvorrichtung 6.1 besprüht. Die Wasser-Latex-Mischung enthält im Ausführungsbeispiel 96 % Wasser und 4 % Latex (Gew.-%). Diese Werte sind nach fachmännischem Ermessen entsprechend dem Typ des verwendeten Latex und der Faserart und Faserverdichtung zu variieren. Der hohe Wasseranteil unterstützt die Bindung zusätzlich, wenn das Wasser verdunstet ist, wie dies von der Papierherstellung aus Fasern bekannt ist. Wesentlich ist weiterhin, dass durch den hohen Wasseranteil in der aufgebrachten Wasser-Latex-Mischung und durch die Filterwirkung der Faserbahn das Latex nur in den Außenbereich der Faserbahn eindringt und somit eine Latexbindung nur für die außen liegenden Faserbereiche eintritt. Das Wasser dringt tiefer in die Faserbahn ein und sorgt für eine Ausbildung oben erwähnter Bindung.

Um das Eindringen der Wasser-Latex-Mischung zu steuern, wird ein Sauggerät 16.1 unterhalb des Förderbandes 13 angeordnet, wobei durch Einstellung des Unterdrucks verschiedene Eindringtiefen erzeugt werden können.

Als Latex wird ein synthetisches Polymer, nämlich ein Ethylen-Vinylacetat-Copolymerisat als wässerige Emulsion, die selbstvernetzende Gruppen aufweist, angewandt (z. B. AIRFLEX; Hersteller Air-Products and Chemical Allentown, PA, USA). Je nach Zusammenhalt der Faserbahn sind 1 bis 5 Gramm Latex in getrocknetem Zustand pro Quadratmeter ausreichend.

Die Trocknung des Wassers erfolgt durch einen kombinierten Infrarot-Warmluft-Trockner 7.1. Die Faserstoffbahn wird anschließend gewendet und optional auch von der Rückseite mit einem Wasser-Latex-Gemisch besprüht (Bezugszahl 6.2).Auch hier kann die Eindringtiefe durch ein Sauggerät 16.2 beeinflusst werden. Auch ist ein weiterer Trockner 7.2 vorgesehen. Anschließend erfolgt das Aufwickeln und Konfektionieren in üblicher Art über eine Wickelvorrichtung 8, 9.

Nach dem Trocken, Ausfällen und Vernetzen des Latex wird praktisch keine Staubbildung durch Fasern, Faserbruchstücke, SAP-Granulate und Staub mehr beobachtet, die aus dem Faserbereich austreten. Zusätzlich erleichtert die Ausrüstung mit Latex ein federndes, nicht so stark zum Knicken neigendes Verhalten der Faserbahn, wie es insbesondere für das Zick-Zack-Legen der Faserbahn erwünscht ist, wobei dies für eine raumsparende Verpackungsform erforderlich ist.

Gegenüber ähnlichen, auf dem Markt befindlichen Produkten ist die Staubbildung um 90 % und mehr herabgesetzt.

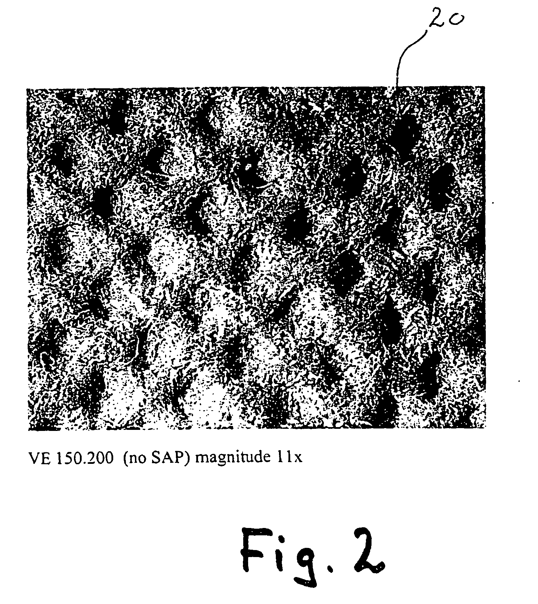

Fig. 2 zeigt in 11facher Vergrößerung dunkle und helle Bereiche einer Faserstruktur, wie sie sich für eine Faserbahn gemäß Erfindung nach den Arbeitsschritten ergibt. Die dunklen Bereiche 20 sind die Eindrücke der Stachelwalze und gleichzeitig Bereiche, in denen sich Latex, das hier dunkel gefärbt ist, an der Oberfläche zeigt. Es ist ersichtlich, dass sich eine flächenhafte, nicht durchgehende Benetzung durch Latex ergeben hat. Die Benetzung ist in sich zusammenhängend, jedoch für bestimmte Flächen, die parallel zur Oberseite liegen, nicht durchgehend, so dass eine gute Saugfähigkeit der Faserbahn erhalten bleibt. Erkennbar sind auch die verdichteten Faser-Verbundbereiche 20, bei denen die Dicke der Faserbahn wesentlich verringert ist.

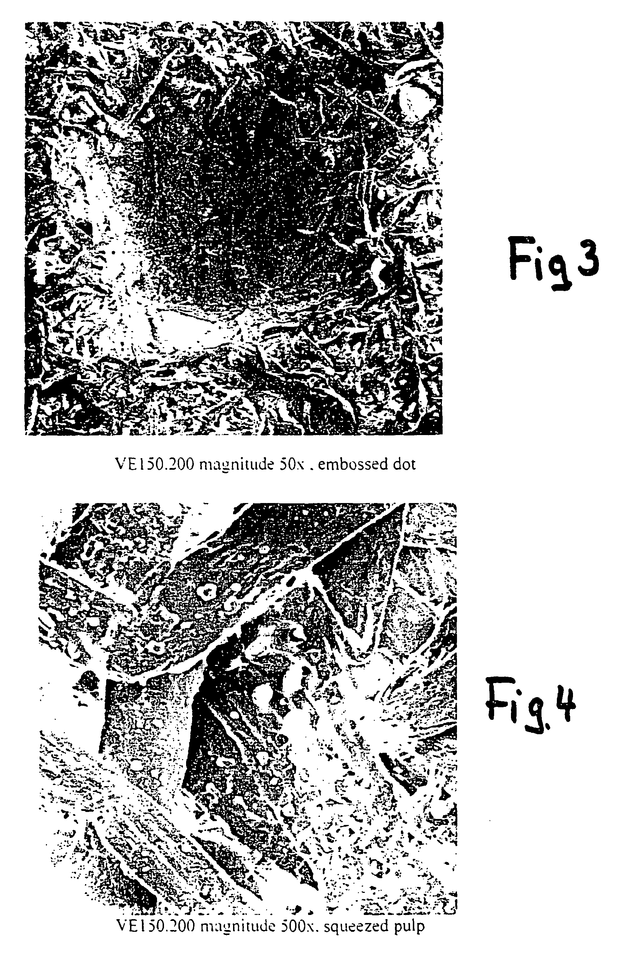

Die Figuren 3 und 4 zeigen rastermikroskopische Aufnahmen der Verbundbereiche 20 aus denen die Eigenbindung der Fasern, die durch den hohen Druck der Walzen gequetscht sind, ersichtlich ist. Figur 3 hat eine 50fache Vergrößerung und zeigt den gesamten Druckpunkt, wogegen Fig. 4 bei 500facher Vergrößerung nur einen Teil eines Verbundbereiches zeigt.

Die bei den Versuchen verwendeten Fasern sind so genannter Fluff Pulp (von Weyerhaeuser oder Tartas). Ähnliche Bilder ergeben sich bei der Verwendung von anderen natürlichen Fasern, wie Baumwolle oder chemisch, thermisch oder mechanisch modifizierter Cellulose, die damit ebenfalls anwendbar sind. Auf den Einsatz von superabsorbierenden Polymeren kann auch verzichtet werden.

Um die Staubabsonderung "linting" mit anderen Produkten zu vergleichen, wurden Vergleichsversuche angestellt. Eine Faserbahn VE 150.200 (ohne SAP) wurde mit einer Faserbahn VE 150.202 (mit SAP) sowie mit zwei Faserbahnen aus Absorptionskernen aus Cellulose-Airlaid-Material verglichen, die aus Unterwäsche-Einlegern, so genannten Slip-Einlagen entnommen wurden. Die nachfolgende Tabelle zeigt die Messergebnisse:

**Tabelle 1**

|  | VE 150.200 | VE 150.202 | Vergl. 1 | Vergl. 2 |
|---|---|---|---|---|
| Flächengew icht in $g/m^2$ | 150 | 150 | 250 | 200 |
| SAP Gehalt (%) | 0 | 15 | 23 | 30 |
| Staubgehalt (%) | 0,0541 | 0,0782 | 0,898 | 0,311 |

Wie aus der Tabelle erkannt wird, wird bei den gemäß Erfindung hergestellten Proben ein niedriger Staubentwicklungsgrad erreicht.

Selbst für den Fall, wo synthetische Polymere als Latex verwendet werden, ist es durch den geringen Gehalt an diesen Polymeren immer noch möglich, eine Kompostierbarkeit zu erreichen. Dies hängt allerdings auch von der Art des verwendeten superabsorbierenden Polymers ab. Auch hier ist es jedoch möglich, SAP einzusetzen, das kompostierbar ist (Markennamen: Lysorb und Sorbfresh (besonders geeignet für Lebensmittel-Saugeinlagen), Hersteller Lysac Technologies Inc., Kanada) .

Die Reißfestigkeit nach Meßmethode EDANA 20.02.89 beträgt nach dem Trocknen etwa 20 N pro 50 mm Bahnbreite, wobei ein Teil der Festigkeit auf den Latex-Auftrag zurückzuführen ist. Als Latices eignen sich wässrige Emulsionen mit Vinylacetat, Acryl-Ester-Polymere, Ethylen-Vinylacetat-Copolymere, Styrol-Butadien-Caproxylat-Copolymere und Polyacrylnitril. Als abbaubare Latices werden solche auf Stärkebasis (z.B.: StructureCote von Vinamul-Polymers) verwendet.

Das Endprodukt hat eine Reißfestigkeit, die es für den Einsatz in Hygieneprodukten geeignet macht. Bei Vergleichsversuchen gemäß folgender Tabelle 2 wurde außerdem festgestellt, dass bei einer Benetzung mit 7 ml Kochsalzlösung mit 0,9 Gew.-% Salzgehalt und Ausbreitung auf einer Fläche mit 200 $cm^2$ eine geringe Rückbefeuchtung (wetback) eintritt. Airlaid Hybrid-Produkte, bei denen die Cellulose-Fasern mit angeschmolzenen Bicomponenten-Fasern gebunden sind und eine Latex-Sprühschicht aufweisen, zeigten eine höhere Rückbefeuchtung. Die bessere Feuchtigkeitsaufnahme der gemäß Erfindung hergestellten Produkte liegt offensichtlich an der nichtdurchgehenden Benetzung durch das bindende Latex.

**Tabelle 2**

|  | VE 150.200 | VE 150.202 | A 150 $g/m^2$ | B 185 $g/m^2$ | C 150 $g/m^2$ |
|---|---|---|---|---|---|
| Bindung | Gem. Erfindung | Gem. Erfindung | Hybrid | Hybrid | Hybrid |
| SAP Gehalt % | 0 | 15 | 15 | 10 | 20 |
| Rückbefe uchtung in Gramm | 0,0108 | 0,0116 | 0,0817 | 0,168 | 0,0536 |

Weitere Beispiele von Anwendungsfällen gehen aus folgender Tabelle 3 hervor, die Mischungsbeispiele für bestimmte Anwendungsfälle zeigt. Dabei soll hervorgehoben werden, dass der Anteil an Latex bei höheren Flächengewichten verringert ist, während der Anteil an angewandtem Wasser im Verhältnis zum getrockneten Endprodukt der gleiche bleibt. Dies wird verwirklicht durch eine stärkere Verdünnung der Latex-Wasser-Mischung. Beispielsweise kann eine Latex-Wasser-Mischung von 6 bis 8 Gew.-% Latex mit 92 bis 94 Gew.-% Wasser in der Version (vgl. Tabelle 3) mit Flächengewicht 120 $g/m^2$ verwendet werden. Bei der Faserbahn mit einem Flächengewicht von 500 $g/m^2$ kann eine Dispersion mit 2 bis 4 Gew.-% Latex aufgesprüht werden. Die Menge des aufgebrachten Wassers ist wichtig, da dann, wenn es verdunstet, das Wasser Wasserstoffbrücken-Bindungen zwischen den Cellulosefasern erzeugt.

**Tabelle 3**

| Anwendungsbeispiele | Flächengewicht in g/m$^2$ (nach Trocknen) | Fasern (fluff pulp) | | SAP, vorzugsweis e in Form von SAF * | | Latex | |
|---|---|---|---|---|---|---|---|
| | | % | g/m$^2$ | % | g/m$^2$ | % | g/m$^2$ |
| Unterwäsche-Einleger; Lebensmittel-Saugeinlagen | 120 | 82 | 98,4 | 15 | 18 | 3 | 3,6 |
| Dünne Hygiene-Saugeinlagen; Lebensmittel-Saugeinlagen | 200 | 68 | 136 | 30 | 60 | 2 | 4 |
| Wegwerfwindeln | 500 | 49 | 245 | 50 | 250 | 1 | 5 |
| *) SAF = superabsorbierende Fasern | | | | | | | |

**Patentansprüche**

1. Verfahren zur Herstellung einer zur Herstellung von Hygiene-Artikeln, wie insbesondere von Inkontinenz-Artikeln, Wegwerf-Windeln, Slipeinlagen oder Monatsbinden, oder Saugeinlagen, geeigneten Faserbahn, deren Faseranteil ausschließlich aus Cellulosefasern natürlichen Ursprungs oder aus Cellulosefasern natürlichen Ursprungs und superabsorbierenden Fasern besteht, mit <u>folgenden Verfahrensschritten:</u>

   - Bildung einer im wesentlichen gleichmäßig dicken, trockenen Faserlegung aus losen Fasern mit einem geringen, im Bereich der Restfeuchte liegenden Feuchtegehalt,
   - Pressen und Prägen der Faserlegung zu einer Faserbahn unter Ausbildung eines Prägemusters mit verdichteten Faser-Verbundbereichen, in denen die Fasern im wesentlichen unter Eigenbindung untereinander verbunden sind,
   - Befeuchten der Faserbahn mit einer Wasser-Latex-Mischung auf wenigstens einer der Außenbereiche, wobei der Einsatz von Latex-Bindern so bemessen ist, dass das Flächengewicht des Latex im Trockenzustand weniger als 5 g/m$^2$ beträgt.
   - Ausfällen des Latex durch Trocknen unter Bindung der Fasern innerhalb und außerhalb der Faser-Verbundbereiche.

2. Verfahren nach Anspruch 1, **dadurch gekennzeichnet, dass** das Flächengewicht der getrockneten Faserbahn auf einen Bereich zwischen 20 und 500 g/m$^2$, vorzugsweise zwischen 100 bis 200 g/m$^2$, eingestellt wird.

3. Verfahren nach Anspruch 1, **dadurch gekennzeichnet, dass** die Ober- und die Unterseite der Bahn in aufeinanderfolgenden Schritten mit der Wasser-Latex-Mischung befeuchtet werden.

4. Verfahren nach Anspruch 1, **dadurch gekennzeichnet, dass** die Wasser-Latex-Mischung 90 bis 99 Gew.-% Wasser und 10 bis 1 Gew.-% Latex enthält.

5. Verfahren nach Anspruch 4, **dadurch gekennzeichnet, dass** die Wasser-Latex-Mischung 92 bis 99 Gew.-% Wasser und 8 bis 1 Gew.-% Latex enthält.

6. Verfahren nach Anspruch 5, **dadurch gekennzeichnet, dass** die Wasser-Latex-Mischung 95 bis 99 Gew.-% Wasser und 5 bis 1 Gew.-% Latex enthält.

7. Verfahren nach Anspruch 1 bis 6, **dadurch gekennzeichnet, dass** während und/oder nach dem Befeuchten der Faserbahn die Durchdringung der Faserbahn mit Hilfe eines an die Faserbahn angelegten Unterdrucks gesteuert wird.

8. Verfahren nach einem der vorhergehenden Ansprüche, **dadurch gekennzeichnet, dass** das Pressen und Prägen der Faserlegung in einer Presswalzenanordnung geschieht, wobei wenigstens eine Walze eine Stachelwalze ist.

9. Verfahren nach Anspruch 1 oder 8, **dadurch gekennzeichnet, dass** je nach Flächengewicht der Faserlegung unterschiedliche Drücke im Bereich von 30 N/mm bis 120 N/mm Liniendruck aufgebracht werden.

10. Verfahren nach einem der vorhergehenden Ansprüche, **dadurch gekennzeichnet, dass** der Faserlegung bzw. der Faserbahn superabsorbierende Polymere (SAP), vorzugsweise als superabsorbierende Fasern, vor dem Pressen und Prägen beigemischt werden.

11. Verfahren nach Anspruch 10, **dadurch gekennzeichnet, dass** die superabsorbierenden Polymere (SAP), vorzugsweise als superabsorbierende Fasern, unter Schichtbildung in die Faserlegung eingebracht werden.

12. Verfahren nach Anspruch 10 oder 11, **dadurch gekennzeichnet, dass** die superabsorbierenden Polymere (SAP) den Cellulosefasern vor der Faserlegung in homogener Verteilung zugemischt werden.

13. Verfahren nach einem der vorhergehenden Ansprüche, **dadurch gekennzeichnet, dass** 16 bis 49 verdichtete Faser-Verbundbereiche pro $cm^2$ der Faserbahn eingebracht sind.

14. Verfahren nach einem der vorhergehenden Ansprüche, **dadurch gekennzeichnet, dass** die verdichteten Faser-Verbundbereiche jeweils eine Fläche von 0,03 bis 1 $mm^2$ abdecken.

15. Verfahren nach einem der vorhergehenden Ansprüche, **dadurch gekennzeichnet, dass** die Wasser-Latex-Mischung mit Hilfe von Walzen als Schaumauftrag oder durch Aufsprühen aufgebracht wird.

16. Verfahren nach einem der vorhergehenden Ansprüche, **dadurch gekennzeichnet, dass** das Abtrocknen des Wassers zum Ausfällen des Latex mit Hilfe von Wärmestrahlung oder mittels Durchblasen von Warmluft durch die Faserbahn erfolgt.

17. Verfahren nach einem der vorhergehenden Ansprüche, **dadurch gekennzeichnet, dass** ein biologisch abbaubares Latex, insbesondere ein Latex auf Stärkebasis, verwendet wird.

18. Verfahren nach einem der vorhergehenden Ansprüche, **dadurch gekennzeichnet, dass** nach Ausfällung und Trocknung der Latex auf wenigstens einer Seite der Faserstoffbahn hydrophil ist.

19. Verfahren nach einem der vorhergehenden Ansprüche, **dadurch gekennzeichnet, dass** für die gegenüberliegenden Seiten der Faserbahn unterschiedliche Latices verwendet werden.

20. Verfahren nach Anspruch 19, **dadurch gekennzeichnet, dass** nach Ausfällung und Trocknung auf der einen Seite der Faserbahn das Latex hydrophil und auf der anderen Seite hydrophob ist.

21. Zur Herstellung von Hygieneartikeln geeignete Faserbahn, hergestellt nach wenigstens einem der Ansprüche 1 bis 20, mit einem Staubgrad unterhalb 0,2 %, gemessen nach einem standardisierten Verfahren.

22. Faserbahn nach Anspruch 21, **dadurch gekennzeichnet, dass** der Anteil an superabsorbierenden Polymeren in der trockenen Faserbahn zwischen 0 und 70 Gew.-% liegt.

23. Faserbahn nach einem der Ansprüche 21 oder 22, **dadurch gekennzeichnet, dass** die Masse des mit der Faserbahn verbundenen Latex im Trockenzustand 1 bis 5 $g/m^2$ beträgt.

**Claims**

1. A method of producing a fibre web which is suitable for the production of hygiene articles, in particular incontinence articles, disposable nappies, panty liners or sanitary towels, and the fibre component of which consists exclusively of cellulose fibres of natural origin or cellulose fibres of natural origin and super-absorbent fibres, comprising the following method steps:

- forming a substantially uniformly thick dry fibre layer of loose fibres with a low moisture content which is in the range of residual moisture,
- pressing and embossing the fibre layer to produce a fibre web with the formation of an embossing pattern with compacted fibre bond regions in which the fibres are substantially connected together with self-bonding,
- wetting the fibre web with a water-latex mixture on at least one of the outer regions, wherein the use of latex bonding agents is such that the weight in relation to surface area of the latex in the dry state is less than 5 g/m$^2$, and
- precipitating the latex by drying with bonding of the fibres within and outside the fibre bond regions.

2. A method according to claim 1 **characterised in that** the weight in relation to surface area of the dried fibre web is set to a range of between 20 and 500 g/m$^2$, preferably between 100 and 200 g/m$^2$.

3. A method according to claim 1 **characterised in that** the top side and the underside of the web are wetted with the water-latex mixture in successive steps.

4. A method according to claim 1 **characterised in that** the water-latex mixture contains 90 to 99% by weight of water and 10 to 1% by weight of latex.

5. A method according to claim 4 **characterised in that** the water-latex mixture contains 92 to 99% by weight of water and 8 to 1% by weight of latex.

6. A method according to claim 5 **characterised in that** the water-latex mixture contains 95 to 99% by weight of water and 5 to 1% by weight of latex.

7. A method according to claims 1 to 6 **characterised in that** penetration of the fibre web is controlled by means of a reduced pressure applied to the fibre web during and/or after wetting of the fibre web.

8. A method according to one of the preceding claims **characterised in that** pressing and embossing of the fibre layer is effected in a pressing roller arrangement, at least one roller being a spiked roller.

9. A method according to claim 1 or claim 8 **characterised in that** different pressures in the range of 30 N/mm to 120 N/mm line pressure are applied depending on the weight in relation to surface area of the fibre layer.

10. A method according to one of the preceding claims **characterised in that** super-absorbent polymers (SAP), preferably in the form of super-absorbent fibres, are added to the fibre layer or the fibre web prior to the pressing and embossing operation.

11. A method according to claim 10 **characterised in that** the super-absorbent polymers (SAP), preferably in the form of super-absorbent fibres, are introduced into the fibre layer, with layer formation occurring.

12. A method according to claim 10 or claim 11 **characterised in that** the super-absorbent polymers (SAP) are added to the cellulose fibres prior to fibre laying in homogeneously distributed relationship.

13. A method according to one of the preceding claims **characterised in that** 16 to 49 compacted fibre bond regions per cm$^2$ of the fibre web are formed.

14. A method according to one of the preceding claims **characterised in that** the compacted fibre bond regions each cover an area of 0.3 to 1 mm$^2$.

15. A method according to one of the preceding claims **characterised in that** the water-latex mixture is applied by means of rolling in the form of a foam application or by spraying.

16. A method according to one of the preceding claims **characterised in that** drying of the water for precipitation of the latex is effected by means of thermal radiation or by means of blowing warm air through the fibre web.

17. A method according to one of the preceding claims **characterised in that** a biologically degradable latex, in particular a starch-based latex, is used.

18. A method according to one of the preceding claims **characterised in that** after precipitation and drying the latex on at least one side of the fibre web is hydrophilic.

19. A method according to one of the preceding claims **characterised in that** different latexes are used for the opposite sides of the fibre web.

20. A method according to claim 19 **characterised in that** after precipitation and drying the latex on one side of the fibre web is hydrophilic and the latex on the other side is hydrophobic.

21. A fibre web suitable for the production of hygiene articles, produced according to at least one of claims 1 to 20, with a degree of dust of below 0.2% measured in accordance with a standardised method.

22. A fibre web according to claim 21 **characterised in that** the proportion of super-absorbent polymers in the dry fibre web is between 0 and 70% by weight.

23. A fibre web according to one of claims 21 and 22 **characterised in that** the mass of the latex bonded to the fibre web in the dry state is from 1 to 5 g/m$^2$.

**Revendications**

1. Procédé pour la fabrication d'une bande de fibres appropriée pour la fabrication d'articles d'hygiène comme notamment des articles pour l'incontinence, des couches jetables, des protège-slips ou des protections périodiques ou bien des buvards, dont la partie en fibres est exclusivement constituée de fibres de cellulose d'origine naturelle ou bien de fibres de cellulose d'origine naturelle et de fibres super absorbantes, présentant les étapes suivantes :

   - la formation d'une couche de fibres sèche et d'une épaisseur sensiblement égale constituée de fibres lâches ayant une teneur en humidité limitée située dans la plage d'humidité résiduelle,
   - le pressage et le matriçage de la couche de fibres sur une bande de fibres par la formation d'un modèle de matriçage avec des zones de liaison des fibres comprimées dans lesquelles les fibres sont reliées entre elles sensiblement par liaison autonome,
   - l'humidification de la bande de fibres avec un mélange d'eau et de latex sur au moins l'une des zones externes, l'emploi de bandes de latex étant mesuré de manière à ce que la masse au mètre carré du latex à l'état sec soit inférieure à 5 g/m$^2$,
   - la précipitation du latex par séchage par la liaison des fibres dans et hors des zones de liaison des fibres.

2. Procédé selon la revendication 1, **caractérisé en ce que** la masse au mètre carré de la bande de fibres séchée est réglée dans la plage comprise entre 20 et 500 g/m$^2$, de préférence entre 100 et 200 g/m$^2$.

3. Procédé selon la revendication 1, **caractérisé en ce que** les parties supérieure et inférieure de la bande sont humidifiées avec le mélange d'eau et de latex lors d'étapes successives.

4. Procédé selon la revendication 1, **caractérisé en ce que** le mélange d'eau et de latex contient 90 à 99 % en poids d'eau et 10 à 1 % en poids de latex.

5. Procédé selon la revendication 4, **caractérisé en ce que** le mélange d'eau et de latex contient 92 à 99 % en poids d'eau et 8 à 1 % en poids de latex.

6. Procédé selon la revendication 5, **caractérisé en ce que** le mélange d'eau et de latex contient 95 à 99 % en poids d'eau et 5 à 1 % en poids de latex.

7. Procédé selon la revendication 1 à 6, **caractérisé en ce que**, pendant et/ou après humidification de la bande de fibres, on dirige la pénétration de la bande de fibres à l'aide d'une dépression exercée sur la bande de fibres.

8. Procédé selon l'une des revendications précédentes, **caractérisé en ce que** le pressage et le matriçage de la couche de fibres s'effectuent dans un arrangement de cylindres presseurs, au moins un cylindre étant un cylindre à picots.

9. Procédé selon la revendication 1 ou 8, **caractérisé en ce que** selon la masse au mètre carré de la couche de fibres, on exerce diverses pressions dans la plage de 30 N/mm à 120 N/mm de pression linéaire.

10. Procédé selon l'une des revendications précédentes, **caractérisé en ce que** des polymères super absorbants (SAP), de préférence sous forme de fibres super absorbantes, sont mélangés à la couche de fibres ou à la bande de fibres avent le pressage et le matriçage.

11. Procédé selon la revendication 10, **caractérisé en ce que** les polymères super absorbants (SAP), de préférence sous forme de fibres super absorbantes, sont introduits dans la couche de fibres par stratification.

12. Procédé selon la revendication 10 ou 11, **caractérisé en ce que** les polymères super absorbants (SAP) sont mélangés aux fibres de cellulose en proportion homogène avant la couche de fibres.

13. Procédé selon l'une des revendications précédentes, **caractérisé en ce que** l'on introduit 16 à 49 zones de liaison des fibres comprimées par $cm^2$ de bande de fibres.

14. Procédé selon l'une des revendications précédentes, **caractérisé en ce que** les zones de liaison des fibres comprimées recouvrent respectivement une surface de 0,03 à 1 $mm^2$.

15. Procédé selon l'une des revendications précédentes, **caractérisé en ce que** le mélange d'eau et de latex est appliqué à l'aide de cylindres en tant qu'application de mousse ou par pulvérisation.

16. Procédé selon l'une des revendications précédentes, **caractérisé en ce que** le séchage de l'eau pour précipiter le latex s'effectue à l'aide d'un rayonnement de chaleur ou en soufflant de l'air chaud à travers la bande de fibres.

17. Procédé selon l'une des revendications précédentes, **caractérisé en ce qu'**on utilise un latex biodégradable, notamment un latex à base d'amidon.

18. Procédé selon l'une des revendications précédentes, **caractérisé en ce qu'**après précipitation et séchage, le latex est hydrophile sur au moins une face de la bande de fibres.

19. Procédé selon l'une des revendications précédentes, **caractérisé en ce qu'**on utilise divers quadrillages pour les faces opposées de la bande de fibres.

20. Procédé selon la revendication 19, **caractérisé en ce qu'**après précipitation et séchage, le latex est hydrophile sur une face de la bande de fibres et hydrophobe sur l'autre face.

21. Bandes de fibres appropriées pour la fabrication d'articles d'hygiène, fabriquées selon au moins l'une des revendications 1 à 20, ayant un degré de poussières inférieur à 0,2 %, mesuré selon un procédé normalisé.

22. Bande de fibres selon la revendication 21, **caractérisée en ce que** la proportion de polymères super absorbants dans la bande de fibres séchée est comprise entre 0 et 70 % en poids.

23. Bande de fibres selon l'une des revendications 21 ou 22, **caractérisée en ce que** la masse du latex fixé à la bande de fibres à l'état sec est de 1 à 5 $g/m^2$.

Fig. 1

EP 1 721 036 B1

20

VE 150.200 (no SAP) magnitude 11x

Fig. 2

Fig 3

VE150.200 magnitude 50x . embossed dot

Fig. 4

VE150.200 magnitude 500x. squeezed pulp

**EP 1 721 036 B1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 9316228 A, Norlander
- WO 8804704 A, Graef
- US 3692622 A